# EUROPEAN PATENT SPECIFICATION

(11) **EP 3 756 428 B1**
(45) Date of publication and mention of the grant of the patent: **18.11.2026**
(21) Application number: 19758124.2
(22) Date of filing: 15.02.2019
(51) Int. Cl.: G01P 13/00, A43D 1/00, A61B 5/11, H02J 50/10, H02J 7/02, H04W 4/38, H04W 84/10, A43B 3/00, A61B 5/00, A61B 5/103

(54) **INSOLE AND INSOLE DOCKING SYSTEM FOR COLLECTING, DOWNLOADING AND ANALYZING GAIT DATA**
EINLEGESOHLE UND EINLEGESOHLEN-ANDOCKSYSTEM ZUM SAMMELN, HERUNTERLADEN UND ANALYSIEREN VON GANGDATEN
SEMELLE INTÉRIEURE ET SYSTÈME D'ACCUEIL DE SEMELLE INTÉRIEURE POUR COLLECTER, TÉLÉCHARGER ET ANALYSER DES DONNÉES DE MARCHE

(30) Priority: 23.02.2018 US 201862634315 P
(43) Date of publication of application: 30.12.2020
(73) Proprietor: Plantiga Technologies Inc., Vancouver, British Columbia V6A 1E1 (CA)
(72) Inventor: SANDLER, Quin, Samuel, McKay, Vancouver, British Columbia V6E 4M8 (CA); FITZGERALD, Colin, Edward, Kimberley, British Columbia V1A 2E6 (CA); RIZUN, Peter, Coquitlam, British Columbia V3J 4Z9 (CA); JAMES, Daryl, Coquitlam, British Columbia V3J 4Z9 (CA)
(74) Representative: Purdylucey Intellectual Property
(86) International application number: PCT/CA2019/000023
(87) International publication number: WO 2019/161480

(56) References cited:
- US-A1- 2003 163 287
- US-A1- 2009 096 413
- US-A1- 2015 257 679
- US-A1- 2020 345 271
- US-B2- 9 655 405
- HEGDE ET AL.: "SmartStep 2.0 - A completely wireless, versatile insole monitoring system", 2015 IEEE INTERNATIONAL CONFERENCE ON BIOINFORMATICS AND BIOMEDICINE (BIBM) , HELD 9- 12 NOV. 2015, 17 December 2015 (2015-12-17), pages 746 - 749, XP032833606
- HEGDE ET AL.: "Development of a real time activity monitoring Android application using SmartStep", 2016 38TH ANNUAL INTERNATIONAL CONFERENCE OF THE IEEE ENGINEERING IN MEDICINE AND BIOLOGY SOCIETY (EMBC), HELD 16- 20 AUGUST 2016, 18 October 2016 (2016-10-18), XP032979538

## Description

### FIELD

The present technology is directed to a system for collecting and analyzing movement data for one or more subjects during a wide range of physical activities. More specifically, it is a system that identifies use of a specific insole and date stamps activity associated with that insole.

### BACKGROUND

Human movement analysis may be performed using signals provided by sensors worn by a test subject on clothing or in the subject's shoes. The sensors provide movement data that can be analyzed to provide information on the test subject's movements with a view to identifying movement problems that may indicate injury or potential for injury, as well as measurements of fatigue, assessment of performance, and the like.

United States Patent No. 9655405 discloses a multilayer insole for removable placement in an article of footwear, the multilayer insole including a bottom insole layer having a top side, a top insole layer having an underside, an intermediate layer that is (a) an insole material layer, (b) a flexible circuit layer or (c) a bonding region for joining the bottom insole layer with the top insole layer, a location data receiving means for receiving an input signal relating to a location of the insole, and a location data transmitting means for transmitting an output signal relating to the location of the insole, wherein the location data receiving means and the location data transmitting means are both integrally associated with the multilayer insole. Also provided are a unitary insole, data transfer systems and methods of using them involving the different insoles and methods of manufacturing the different insoles. Charging can be done using a USB cord or by a wireless magnetic induction system. It does not permit charging and using a plurality of insoles, each pair on a different user, in a trackable manner. Hence, study of subjects is slow and limited to the study of one subject at a time.

United States Patent Application 20110275956 discloses an intelligent insole for generating time sensitive information about the pressure on the foot. The insole includes a custom-made, semi-custom or generically sized orthotic component. The orthotic is laminated with a top cover and an intermediate pressure sensor having an array of capacitive pressure sensors. Signal processing equipment may be embedded in the insole or placed locally with the insole as on the side of a shoe. The processor also can connect to a wireless transmitter for relaying the information to a remote site. Charging can be done using a battery or by a wireless magnetic induction system in the insole. It does not permit charging and using a plurality of insoles, each pair on a different user, in a trackable manner. Hence, study of subjects is slow and limited to the study of one subject at a time.

United States Patent Application 20160066644 discloses a locating and tracking system that includes shoes, which carry a wireless module. A user walking the area causes clicking or intermittent energizing a transmitter carried in the module. The transmitter communicates with a plurality of fixed wireless access points, to provide the position and tracking for persons in the area. This system is limited to position tracking of one person at a time. It does not permit charging and using a plurality of insoles, each pair on a different user, in a trackable manner. Further, it is not directed to motion studies.

Hedge Nagaraj et al., IEEE International conference On Bioinformatics and Biomedicine (BIBM), IEEE, pp. 746-749 (2015) describes a wireless, versatile gait data acquisition insole monitor.

US 2003/163287 A1 describes a smart sensor (adhesive bandage) comprising internal detectors that sense conditions associated with movement or the environment of the sensor and wirelessly communicates with remote receivers.

What is needed is at least one insole includes a power source, processor and memory and that collects and stores data from sensors such as pressure sensors, inertia sensors and the like that report on foot movement, gait, foot pressure and the like. It would be preferable if the memory stored the resultant data sets. It would be still more preferable if the data sets were downloaded onto a dock in a docking system, which concomitantly charged the power source. It would be further preferable if individual insoles in a plurality of insoles could be temporally and spatially tracked. It would be still preferable if the docking system either stored and analyzed the data sets or sent the data sets to a remote computing system, which could be in a cloud, for data analysis and storage.

### SUMMARY

The present technology provides at least one insole that includes a power source, processor and memory and that collects and stores data from sensors such as pressure sensors, inertia sensors and the like that report on foot movement, gait, foot pressure and the like. The memory stores the resultant data sets, which are then downloaded onto a dock. The dock also charges the power source. Individual insoles in a plurality of insoles can be temporally and spatially tracked. The dock may be one of a plurality of docks on a docking system. The docking system either stores and analyzes the data sets or sends the data sets to a remote computing system, which may be in a cloud, for data analysis and storage.

There is provided a system for collecting, storing and analyzing movement data associated with physical activity as set out in the appended claims.

There is also provided a method of collecting and storing movement data as set out in the appended claims.

In one embodiment not covered by the subject-matter of the claims, a system comprising at least one dock is provided, the dock for use with a computer, and at least one insole, the insole for use with an energy storage device, the insole including: circuitry, which includes a memory, a processor, the processor under control of the memory, and a switch, the switch under control of the processor; at least sensor which is at least one of a motion sensor and a pressure sensor, the sensor in electronic communication with the processor; at least one magnet or magnetic material which is in electronic communication with the processor; a charger for electrical communication with an energy storage device; an electronic communicator, which is in electronic communication with the circuitry and is configured for electronic communication with the dock; and a substrate, which retains the circuitry, the sensor, the magnet or magnetic material, the charger and the electronic communicator, the dock including at least one magnet or magnetic material, for providing a magnetic field between the insole and the dock and an electronic communicator for electronic communication with the insole circuitry and for electronic communication with a computer.

In the system not covered by the subject-matter of the claims, the insole may further comprise a time and date stamper, which is retained by the substrate and is in electronic communication with the memory and the processor.

In the system not covered by the subject-matter of the claims, the memory of the insole may be configured to store data from the sensor as a data set.

In the system not covered by the subject-matter of the claims, there may be at least two insoles, a first insole including a first polarity magnet, and a second insole including a second polarity magnet, the first polarity magnet and the second polarity magnet for providing a magnetic attraction.

In the system not covered by the subject-matter of the claims, there may be at least two docks, a first dock including a first polarity dock magnet and the second dock including a second polarity dock magnet, the first polarity magnet and the second polarity dock magnet providing a magnetic attraction and the second polarity magnet and the first polarity dock magnet for providing a magnetic attraction.

In the system not covered by the subject-matter of the claims, the memory may be configured to instruct the processor, in response to a loss of the magnetic attraction, to activate the circuitry with the switch.

In the system not covered by the subject-matter of the claims, the first insole electronic communicator may be a first wireless antenna or transceiver with a first data channel, the second insole electronic communicator may be a second wireless antenna or transceiver with a second data channel and the first dock electronic communicator and the second dock electronic communicator may be wireless transceivers.

The system not covered by the subject-matter of the claims may further comprise a kiosk, the kiosk housing the docks.

In the system not covered by the subject-matter of the claims, the kiosk may include the computing device, the computing device in electronic communication with the docks.

In the system not covered by the subject-matter of the claims, the computer may be configured to store and analyze the data set to provide an analyzed data set.

In the system not covered by the subject-matter of the claims, the docks may each include a charging module for electrical communication with the chargers of the insoles.

In the system not covered by the subject-matter of the claims, the insoles may include the energy storage device.

In the system not covered by the subject-matter of the claims, there may be a plurality of first insoles, a plurality of second insoles, and a plurality of docks.

In an embodiment covered by the subject-matter of the claims, a system for collecting, storing and analyzing movement data associated with physical activity is provided, the system comprising at least one dock and at least one insole, the insole for use with an energy storage device, the insole including: circuitry, which includes a memory, a processor, the processor under control of the memory and a first polarity magnet or magnetic material; the at least one dock, which includes a second polarity magnet or a magnetic material which is magnetically attracted to the first second polarity magnet or magnetic material of the insole; at least one sensor, which is at least one of a motion sensor and a pressure sensor and which is in electronic communication with the processor; a charger for electrical communication with an energy storage device; an electronic communicator, which is in electronic communication with the circuitry and is configured for electronic communication with the dock; and a substrate, which retains the circuitry, the sensor, the charger and the electronic communicator, the dock including an electronic communicator for electronic communication with the insole and for electronic communication with a computer, wherein the memory is configured to instruct the processor to collect data from the sensor to provide a data set; configured to store the data set; and configured to instruct the processor, in response to the magnetic attraction between the first polarity magnet or magnetic material and the second polarity magnet or magnetic material, to download the data set to the dock.

In the system, the electronic communicators may be a wireless interface.

The system may comprise at least a first insole and a second insole, the wireless interface of each insole having a discrete data channel.

In the system, the memory may be further configured to send the data set periodically during data collection via the discrete data channel of each insole to the dock.

The system may further comprise a computing device, the computing device including a device processor and a device memory, the computing device in electronic communication with the dock.

In the system, the device memory may be configured to instruct the device processor to analyze the data set to provide an analyzed data set.

In the system, the device memory may be configured to store the analyzed data set.

In the system, the device memory may be configured to instruct the processor to develop a predictive model based on the analyzed data set.

In yet another embodiment covered by the subject-matter of the claims, a method of collecting and storing movement data is provided, the method comprising:
- a user selecting at least one insole from a dock, the insole including circuitry, which includes a memory, a processor, the processor under control of the memory, and a first polarity magnet or magnetic material; the dock including a communications interface and a second polarity magnet or magnetic material which is magnetically attracted to the first polarity magnet or magnetic material; at least one sensor, which is at least one of a motion sensor and a pressure sensor and which is in electronic communication with the processor; an energy storage device, which is in electrical communication with the circuitry; a charger in electrical communication with the energy storage device; an insole communications interface, which is in electronic communication with the circuitry and the dock; and a substrate, which retains the circuitry, the sensor, the charger and the communications interface;
- the user removing the insole from the dock and releasably retaining the insole on the user's foot;
- the processor signaling the memory to start data collection;
- the sensor sending data to the circuitry, in response to a magnetic attraction between the first polarity magnet and the second polarity magnet, to provide a data set; and
- the memory storing the data set.

In the method, the removing the insole from the dock may break a magnetic field between the first polarity magnet or magnetic material of the insole and the second polarity magnet or magnetic material of the insole and the dock for causing a switch under the control of the processor in the insole to start data collection.

The method may further comprise:
- the user removing the insole and returning it to the dock resulting in a magnetic field between the first and second polarity magnets or magnetic materials of the insole and the dock, respectively, for causing a switch to start of data downloading from the insole to the dock.

The method may further comprise:
- the dock transmitting the data set to a computer; and
- the computer analyzing the data set to provide an analyzed data set.

In the method, the analyzing may provide a predictive model.

In another embodiment not covered by the subject-matter of the claims, a system comprising at least one dock is provided, the dock for use with a computer, and at least one insole, the insole for use with an energy storage device, the insole including: circuitry, which includes a memory, a processor, the processor under control of the memory, and a switch, the switch under control of the processor; at least sensor which is at least one of a motion sensor and a pressure sensor, the sensor in electronic communication with the processor; a charger for electrical communication with an energy storage device; and a substrate, which retains the circuitry, the sensor and the charger, wherein when the insole is located on the dock, there is a magnetic field between the insole and the dock and a communication interface between the insole and the dock and when the insole is removed from the dock, the magnetic field is broken.

In another embodiment not covered by the subject-matter of the claims, a method of collecting and storing movement data is provided, the method comprising:
- a user selecting at least one insole from a dock, the dock including a communications interface, the insole including circuitry, which includes a memory, a processor, the processor under control of the memory; at least one sensor, which is at least one of a motion sensor and a pressure sensor and which is in electronic communication with the processor; an energy storage device, which is in electrical communication with the circuitry; a charger in electrical communication with the energy storage device; an insole communications interface, which includes discrete data channels and which is in electronic communication with the circuitry and the dock; and a substrate, which retains the circuitry, the sensor, the charger and the communications interface;
- the user removing the insole from the dock and releasably retaining the insole on the user's foot;
- the processor signaling the memory to start data collection;
- the sensor sending data to the circuitry, to provide a data set;
- the memory transiently storing the data set;
- the wireless transmitter or transceiver transmitting the data set to the dock or the kiosk in real time via the discrete data channels.

The embedded circuitry within the insole may include at least one sensor operably configured to generate a signal in response to motion of the insole while being worn by a user, and an insole controller operable to receive the signal from the sensor and to wirelessly transmit data representing motion of the insole to a host controller.

The externally accessible contacts of the dock connector may further include at least one contact operably configured to carry a wired communication signal for interfacing between the charging apparatus and a host controller.

In accordance with another disclosed aspect not covered by the subject-matter of the claims there is provided a kiosk system for accommodating a plurality of pairs of removable insoles, each insole having an energy storage device, embedded circuitry, and an embedded receiving antenna, the embedded circuitry being operably configured to sense and record movement of a shoe in which the insole is inserted. The kiosk system includes a storage area having a plurality of locations for storing pairs of insoles, each location including a charging apparatus having an interface surface and a transmission antenna disposed proximate the interface surface, the transmitting antenna being coupled to an antenna driver operable to cause the antenna to generate an alternating magnetic field for charging the energy storage device when the insole is received on the interface surface. The system also includes a host controller having a wireless interface operably configured to wirelessly connect to the embedded circuitry in the insole for receiving movement information from the insole while being worn in a test subject's shoes during a movement test, and an interactive display operable to display a user interface and receive input information related to the test subject, the display being in communication with the host controller for transferring the input information to the host controller.

The embedded circuitry in the insole may include a plurality of inertial sensors that generate signals representing movement of the insoles worn in the test subjects shoes and the wireless interface of the host controller implements a data protocol having sufficient bandwidth to receive continuous movement information from each of the plurality of inertial sensors in each of the insoles during the movement test.

The embedded circuitry in the insole may include a wireless interface and a buffer memory for accumulating movement information and the wireless interface of the insole may be operably configured to periodically transmit movement information to the wireless interface of the host controller from the buffer memory, following which the wireless interface of the insole is placed in a standby mode to conserve energy.

The wireless interface of the host controller and the wireless interface of the insole further implement a low power wireless protocol for transfer of data other than the movement data between the embedded circuitry in the insole and the host controller.

The host controller may further include a communications interface in communication with a computer network for transferring movement information received from the insoles to a networked computing resource operable to receive and process the movement data and to make movement test results available to the test subject.

The host controller may be operably configured to receive the movement test results at the communications interface and cause the results to be displayed on the interactive display.

The communications interface of the host controller further implements a protocol for wired communication with the respective charging apparatuses associated with each location to determine a status associated with the location.

The status associated with each location may include at least one of whether an insole may be present at the location, a size of the insole present at the location, and a state of charge of insoles stored at the location.

In accordance with another disclosed aspect not covered by the subject-matter of the claims there is provided a method for performing a movement test on a test subject wearing a pair of shoes having insoles therein, each insole having an energy storage device, embedded circuitry, and an embedded receiving antenna, the embedded circuitry in the insole being operably configured to sense and record movement of a shoe in which the insole is inserted, the insoles being selected from a plurality of different pairs of insoles stored in a kiosk. The method involves receiving an identification of the selected insoles at an interactive display in communication with a host controller associated with the kiosk, receiving information identifying the test subject via the interactive display, causing the host controller to make an association between the selected insoles and the test subject, receiving movement data from the insoles during the movement test at a wireless interface of the host controller, generating a movement test record including the movement data and the information identifying the test subject, and analyzing the test record to generate movement test results.

Analyzing the test record may involve transmitting the test record via a communications interface of the host controller to a networked computing resource, the networked computing resource may be operably configured to receive and process the movement data and to make movement test results available to the test subject.

### BRIEF DESCRIPTION OF THE DRAWINGS

In drawings which illustrate disclosed embodiments,
- Figure 1: is a perspective view of a charging;
- Figure 2: is a perspective view of a shoe and insole for use with the charging apparatus shown in Figure 1;
- Figure 3: is a further perspective view of the charging apparatus shown in Figure 1 with an upper portion of the housing removed;
- Figure 4: is a perspective view of the charging apparatus shown in Figure 1 having a pair of shoes disposed for charging;
- Figure 5: is a block diagram of components located within the housing of the charging apparatus shown in Figure 3;
- Figure 6: is a further perspective view of a base portion of the charging apparatus shown in Figure 1;
- Figure 7: is a perspective view of a charging mat;
- Figure 8: is a further perspective view of the charging mat of Figure 7;
- Figure 9: is a schematic of the insole of the present technology;
- Figure 10: is a block diagram of the components of a plurality of insoles and their communication with the kiosk system;
- Figure 11: is a perspective view of a plurality of charging mats as shown in Figures 7 and 8 disposed in a side-by-side arrangement;
- Figure 12: is a perspective view of a kiosk system kiosk system;
- Figure 13: is a block diagram showing components of a host controller of the kiosk system shown in Figure 12;
- Figure 14: is a block diagram of an insole; and
- Figure 15: is a process flowchart shown blocks of codes for directing a processor circuit of the host controller shown in Figure 13 to conduct a movement test.

### DETAILED DESCRIPTION

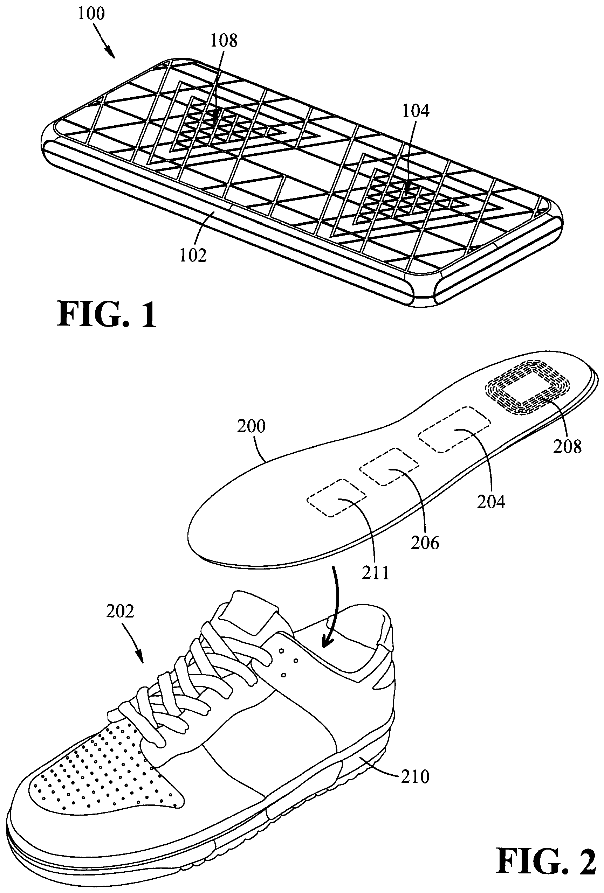

A charging apparatus for wirelessly charging an energy storage device within a shoe is shown at 100 in Figure 1. The charging apparatus 100 includes a housing 102 having at least one interface surface 104 sized to receive at least a portion of a sole of the shoe. Referring to Figure 2, the energy storage device is embedded within an insole 200 of a shoe 202 as shown in Figure 2. The insole 200 includes an energy storage device 204, which may be a battery such as a lithium polymer battery, supercapacitor, or other energy storage technology. The insole 200 also includes embedded circuitry 206 and an embedded receiving antenna 208. The embedded circuitry may include a wireless interface 211, which may be implemented as an IEEE 802.11 Wi-Fi interface and/or a Bluetooth interface. The energy storage device 204 is operable to power the embedded circuitry 206 within the insole for sensing movement of the shoe 202 when the insole is inserted in the shoe. The energy storage device 204, circuitry 206, and receiving antenna 208 may be otherwise embedded or attached to the shoe 202. For example, the receiving antenna 208 may be embedded within a heel portion 210 of the shoe 202 or attached to the inside of the shoe under the insole. The insole 200 as disclosed in commonly owned PCT application WO2018/170581 entitled "COMPACT UNDER-FOOT DEVICE THAT MEASURES THE MOTION OF EACH PART OF THE FOOT", filed on March 23, 2018.

While a single shoe 202 is shown in Figure 2, each shoe of a pair of shoes may have an insole 200 having a respective energy storage device 204, circuitry 206, and receiving antenna 208. In Figure 1, the charging apparatus 100 includes the interface surface 104 for receiving a left shoe 202 and also includes a second interface surface 108 for receiving a right shoe. The charging apparatus 100 may have only a single interface surface 104.

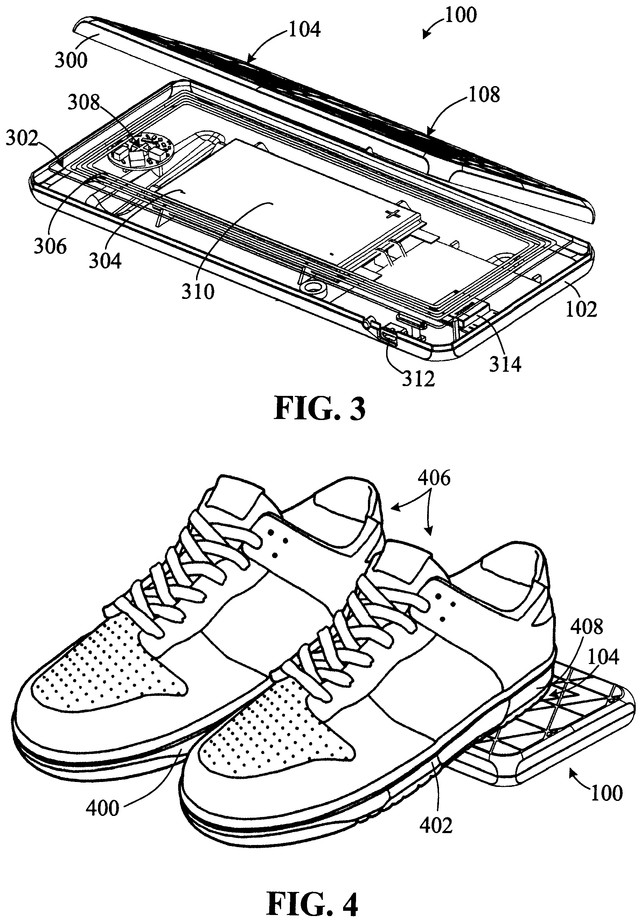

The charging apparatus 100 is shown in Figure 3 with an upper portion 300 of the housing 104 removed to reveal components within the housing 102. Referring to Figure 3, the charging apparatus 100 includes a transmission antenna 302 disposed within the housing 102 proximate the interface surfaces 104 and 108. The transmission antenna 302 comprises a multi-layer multi-mode antenna manufactured by NuCurrent^{®} of Chicago IL, which includes a planar flexible insulating substrate 304 having an inductor coil 306 formed on the substrate. The transmission antenna 302 is driven by an antenna driver circuit 308 coupled to the transmission antenna. The antenna driver 308 is operable to cause the antenna 302 to generate an alternating magnetic field for charging the energy storage device 204 in the shoe 202 when the shoe is received on the interface surface 104. The alternating magnetic field generated by the transmission antenna 302 induces an alternating current in the receiving antenna 208 embedded within the insole 200 of the shoe 202, thus transferring energy between the charging apparatus 100 and receiving antenna for charging the energy storage device 204. The wireless charging may be implemented to comply with one of the common wireless charging interface standards such as Qi (Wireless power consortium) or Rezence (AirFuel Alliance).

The charging apparatus 100 also includes a battery 310 (shown under the substrate 304 of the transmission antenna 302). The charging apparatus 100 also includes an externally accessible charge connector 312 operable to receive a charge current for charging the battery 310. The battery 310 provides power to the antenna drive circuit 308 for generating the alternating magnetic field. The battery 310 may have a capacity greater than the capacity of the energy storage device 204 associated with the insole 200 so that the charging apparatus 100 is capable of at least fully charging the energy storage device 204 in the insole 200. Typically the battery 310 will have sufficient capacity for several full charges and in one embodiment the battery 310 may have a capacity of about 4000 mAh, suitable for charging the energy storage device 204 many times. The externally accessible charge connector 312 may be implemented as a commonly available universal serial bus (USB) type connector, such as a USB Micro B connector, which has been adopted broadly by industry as a universal charging connector.

Referring to Figure 4, the interface surfaces 104 and 108 shown in Figure 1 are sized and spaced apart to receive respective portions of soles 400 and 402 of a pair of adjacently disposed shoes 406, each shoe 406 having an insole 200 located within the shoe. The transmission antenna 302 in the charging apparatus 100 is operably configured to simultaneously induce alternating current in each of the receiving antennas 204 of the respective shoes when a heel portion (for example a portion 408 of the sole 402 of the left shoe 406) is resting on and generally aligned with either if the interface surfaces 104 and 108. The charging apparatus 100 may have a single interface surface 104 for receiving a single shoe 406 and the transmission antenna 302 may be appropriately sized to couple with only a single receiving antenna 208. The receiving antenna 208 may alternatively be disposed in a portion of the sole 402 of the shoe other than the heel portion 408 and the charging apparatus 100 may be configured to receive this portion of the shoe. In general, the energy storage device 204 in the insole 200 may be charged while the insole 200 is inserted in the shoe 202 or the insole 200 may be removed and placed on the interface surface 104 of the charging apparatus 100 for charging.

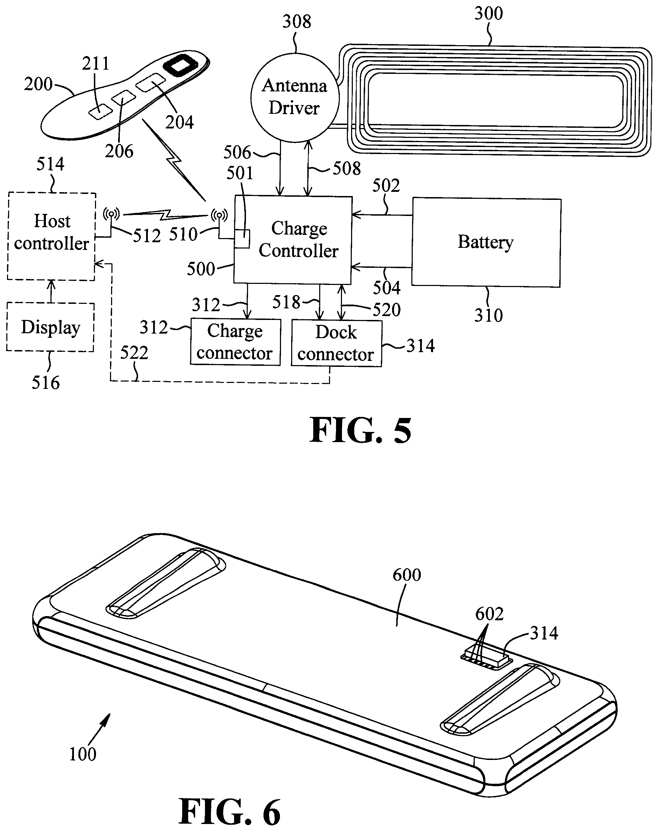

A block diagram of components of the charging apparatus 100 within the housing 102 is shown in Figure 5. Referring to Figure 5, the transmission antenna 302 is coupled to the antenna driver 308 for generating the magnetic field as described above. The charging apparatus 100 also includes a charge controller 500, which is connected to the battery 310 via a power connection 502 and a signal line 504. The power connector line 502 either delivers power from the battery to the controller 500 or carries a charging current from the controller when the battery 310 needs charging. The signal line 504 carries a signal for delivering battery status information to the controller 500 such as state of charge and/or battery temperature information. When a charge current is received by the charge controller 500 via the charge connector 312 the charge controller controls charging of the battery 310. The charge controller 500 also supplies power to the antenna driver 308 via a power connection 506 and may receive and/or transmit status information and commends over a signal line 508. The status information includes, but is not limited to charging, fully charged, partially charged, not charged, and not charging.

The charge controller 500 may be implemented using an embedded microprocessor circuit 501 that further includes a wireless interface 510. The wireless interface 510 may be implemented as an IEEE 802.11 Wi-Fi interface and/or a Bluetooth interface. The charge controller 500 may be operably configured to wirelessly connect via the wireless interface 510 to a wireless interface 512 of an optional external host controller 514 for providing status information associated with the charging operations of the charging apparatus 100. As noted above, the charge controller 500 may collect status information such as a state of charge of the battery 310 and/or power transfer information representing power transfer to a shoe 406 being currently charged. This information may be externally communicated via the wireless interface 510 to the wireless interface 512 of the host controller 514 for display to a user on a display 516 connected to the host controller 514.

In other embodiments, the wireless interface 211 in the embedded circuitry 206 in the insole 200 may send information to the charge controller 500 related to a state of charge of the energy storage device 204 and/or other information from the shoe being currently charged. For example, other information such as battery usage information may also be received from the embedded circuitry 206 in the insole 200.

Referring back to Figure 3, in the embodiment shown the charging apparatus 100 also includes a dock connector 314. The dock connector 314 may be used as an alternative for connecting charging current to the charging apparatus 100. As shown in Figure 5, the dock connector 314 is connected to the controller 500 via a power connection 518 for delivering power to the controller. The dock connector may also include one or more signal lines 520 for interfacing with a host such as the external host controller 514 over a wired connection. For example, the dock connector 314 may optionally connect to the external host controller 514 via a RS-485 serial communications line 522.

Referring to Figure 6, the charging apparatus 100 is shown with a base portion 600 oriented upwardly. The dock connector 314 is externally accessible on a base 600 of the charging apparatus 100. The dock connector 314 includes a plurality of accessible contacts 602 for delivering charging current to the charging apparatus 100 and for carrying the communication signals between the charging apparatus 100 and the external host controller 514. In general, current for charging of the battery 310 may be selectively provided via either the dock connector 314 or the charge connector 312 depending on which is connected to receive the charge current.

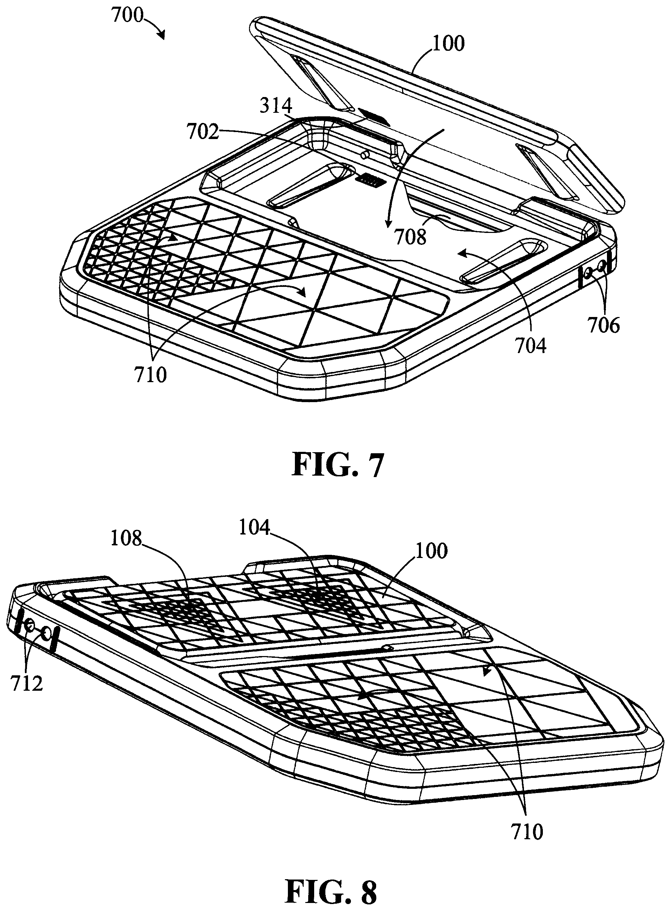

Referring to Figure 7 the charging apparatus 100 may be received in a charging mat shown generally at 700. The charging mat 700 includes a docking port 702 located within a recess 704, the recess being sized to receive the charging apparatus 100. When received in the recess 704 the dock connector 314 on the charging apparatus 100 connects to the docking port 702. The charging mat 700 further includes a first connector 706, which is connected to the docking port 702 via an internal printed circuit board 708 (shown in cut away view). The first connector 706 facilitates connection of a charging current, which is carried via the printed circuit board 708 to the docking port 702. The charging mat 700 further includes an upwardly oriented support surface 710 for supporting the sole of the shoe, or a pair of adjacently disposed shoes. The recess 704 is disposed within the support surface 710 and when the charging apparatus 100 is received in the recess as shown in Figure 7, the interface surfaces 104 and 108 will be substantially aligned with the embedded receiving antennas 208 to support a pair of shoes on the charging mat 700. As shown in Figure 8, the charging mat 700 further includes a second connector 712, substantially identical to the first connector 706. The second connector 712 is also connected to the docking port 702 in parallel with the first connector via the printed circuit board 708. The first connector 706 is disposed on a first side of the charging mat 700 and the second connector 712 is disposed on a second side of the charging mat.

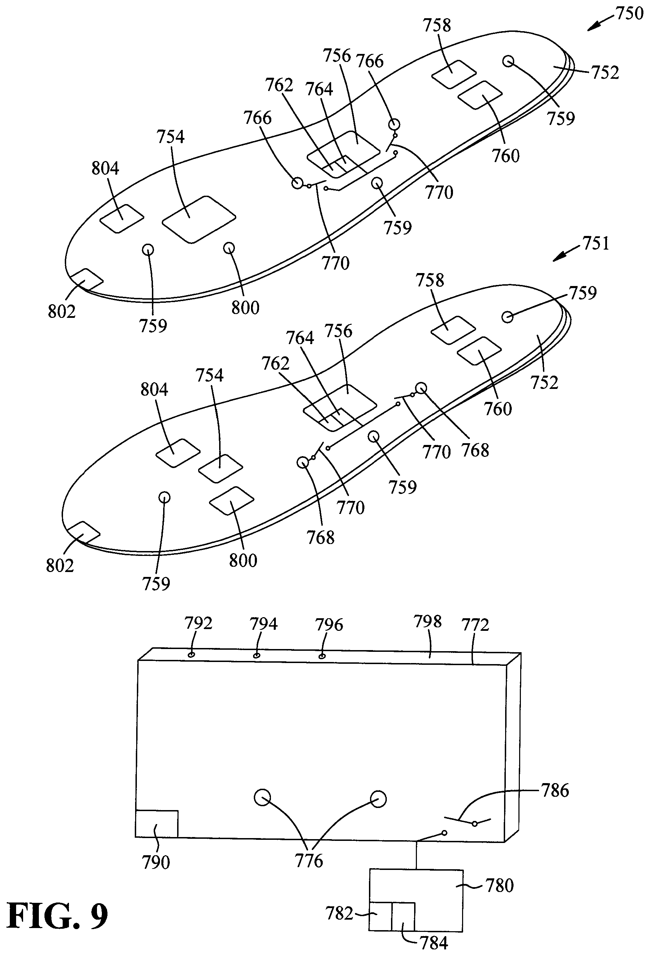

In Figure 9, there is illustrated an insole, generally referred to as 750, 751 includes a substrate 752 which has the general shape of a user's sole and can replace an insole in a shoe. The substrate 752 retains the energy storage device 754, embedded circuitry 756, a charger 758, a plurality of sensors 759 and a wireless interface 760, which may be implemented as an IEEE 802.11 Wi-Fi interface and/or a Bluetooth interface. The charger 758 may be an electrical connection, a USB connection or a receiving antenna. The embedded circuitry 756 in the insole 750, 751 includes a processor 762 under control of a memory 764. The memory 764 has instructions thereon for controlling the processor 762 and is configured to accumulate movement information from the sensors 759. The insole 750, 751 further includes at least one magnet 766 and preferably two, which are retained in the substrate 752. One insole 750 has a first polarity magnet 766 while the other insole 751 has a second polarity magnet 768, or vice versa, such that the magnets are attracted to one another. The processor 762 is in electronic communication with the magnets 766, 768 and in electronic communication with a switch 770. Similarly, a dock 772 has at least one magnet 776 and preferably two, that are the reverse polarity to that of the magnets 766, 768 of the insole 750, 751. The dock magnet 776 is in communication with a host controller 780, which includes a host microprocessor 782 and a host memory 784. The host microprocessor 782 is also in electronic communication with a dock switch 786. The insole processor 762 and memory 764 are also in electronic communication with the host controller 780 via an electronic communicator 800, which is retained on the substrate 752. This electronic communicator 800 may be wireless, for example, but not limited to WIFI or Bluetooth^{®} radio and therefore is the wireless interface 760. The electronic communicator 800 may alternatively be via a wired connection 802, for example, but not limited to a USB connection. A time and date stamper 804 in the insole 750, 751 is in electronic communication with the processor 762 and memory 764. It is retained on the substrate 752. The time and date stamper 804 reports when an individual insole is used, so for example, if a test subject wears only one insole on day one, and then wears the other insole on day two, the data from day one will be reported as being separate from the data from day two.

The insoles 750, 751 may be worn with a range of footwear, for example, but not limited to, shoes, runners, boots or sandals, or in garments such as, but not limited to, socks and stockings.

The insoles may be integral with the footwear or garments. The insoles are provided as separate components for use with the footwear or garments.

The dock 772 further includes a charging module 790, which may be a magnetic induction charger or a USB charger, or an electrical connection or the like, as described above. As would be known to one skilled in the art, the insole 750, 751 is also provided with the corresponding charger 758, which is a receiving antenna, a USB port or an electrical outlet.

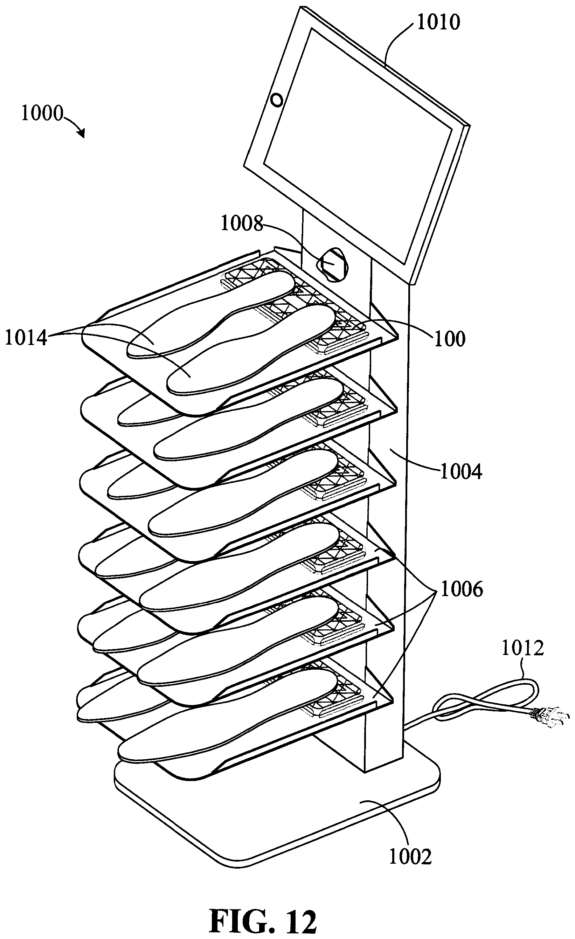

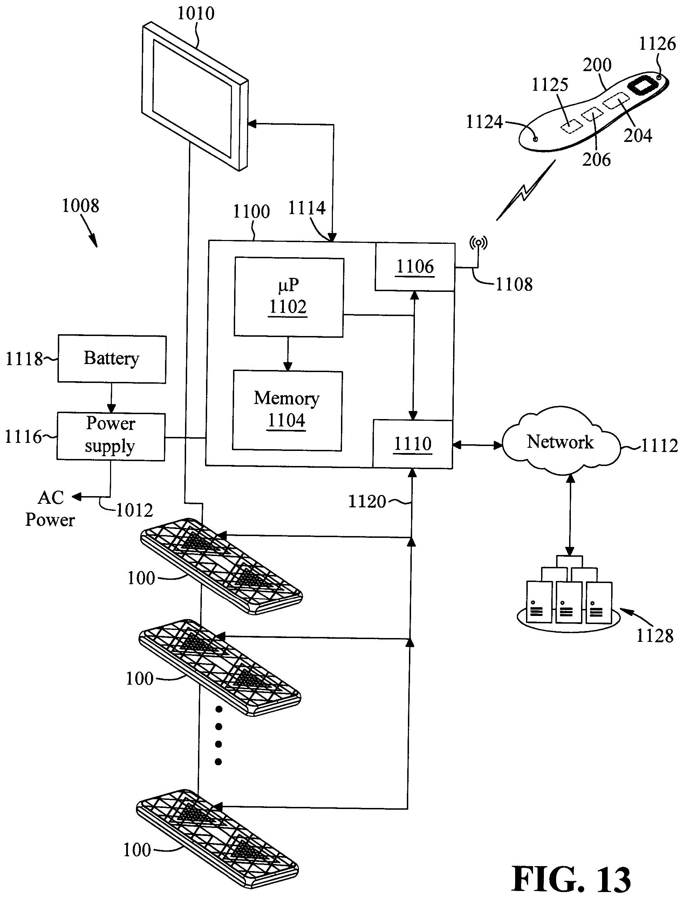

Three lights 792, 794, 796 are located on an outer surface 798 of the dock 772 and are in communication with the host microprocessor 782. A first light 792 may, for example, indicate that the dock 772 is connected to the host microprocessor 782. The second light 794 may, for example, indicate that the insole 750, 751 is connected to and communicating with the microprocessor 782 via the dock 772. The third light 796 may, for example, indicate that the insole 750, 751 is charging, in other words, the energy storage device 754 is in electrical communication with the charging module 790. As shown in Figure 12, a kiosk system 1000 retains a plurality of docks 772. It also retains the host microprocessor 782, which in Figure 13 is referred to as microprocessor 1102. The electronics and charging system of the kiosk system 1000 is the docking system.

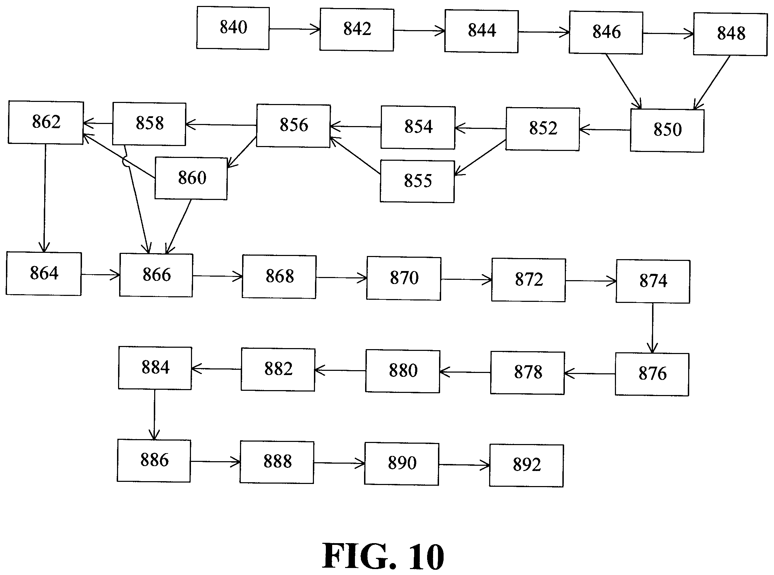

The steps of using the system of Figure 9 is shown in Figure 10. At a first step, the user identification and metrics, as needed, is entered 840 into the host controller 1008 and associated 842 with a pair of insoles. The insoles are magnetically attached 844 to the dock and are fully charged. The magnetic connection is broken 846. The insoles are optionally magnetically connected 848 to one another until they are placed 850 in a user's shoes. Once the magnetic connection is broken, the switch is tripped 852 and the sensors send 854 sensing data to the insole processor and insole memory. The time and date stamper also send 855 time and date data to the memory. The insole memory stores 856 the data. Data collection continues until either the energy storage device is depleted 858 or the insoles are removed 860 from the shoe and are magnetically attached to one another 862 and then magnetically attached 864 to the dock or are directly magnetically attached 866 to the dock. The third light on the dock is illuminated 868 to indicate that the dock is charging 870 the insole. The first light on the dock is illuminated 872 to indicate that the data are downloading 874 onto the host microprocessor. The first light turns off 876 to indicate that the data download has been completed 878. The third light turns 880 to off to indicate 882 that the insole is fully charged. The data are stored 884 as raw data on the host microprocessor and are analyzed 886 and stored 888 as analyzed data in the host microprocessor. The analyzed data includes, but is not limited to, change in gait over time during a single session, change in gait over time, for example over months, change in gait during treatment and change in gait during disease or condition progression, including aging. Other metrics include gait characterization in relation to specific conditions or diseases, gait characterization for specific sports and changes in gait during training. Still further applications include acceleration at the foot, and the asymmetry and distribution of those forces in any given moment, as well as the right versus left of those acceleration forces or pressures. Predictive models are developed 890. Once the insoles are charged they are ready to be removed 892 from the dock and used again. Note that one insole can be used at a time, one pair of insoles can be used at a time and multiple single or pairs of insoles can be used at any given time.

The wireless interface 760 in the insole 750, 751 may be operably configured to periodically transmit movement information to the wireless interface 1106 of the controller unit 1100 from the memory 764, following which the wireless interface 760 of the insole 750, 751 may be placed in a standby mode to conserve energy. IEEE 802.11 communications, while providing high transmission bandwidth, consume more energy than Bluetooth communications and if permitted to continuously transmit movement data may end up draining the energy storage device 754 of the insole 750, 751.

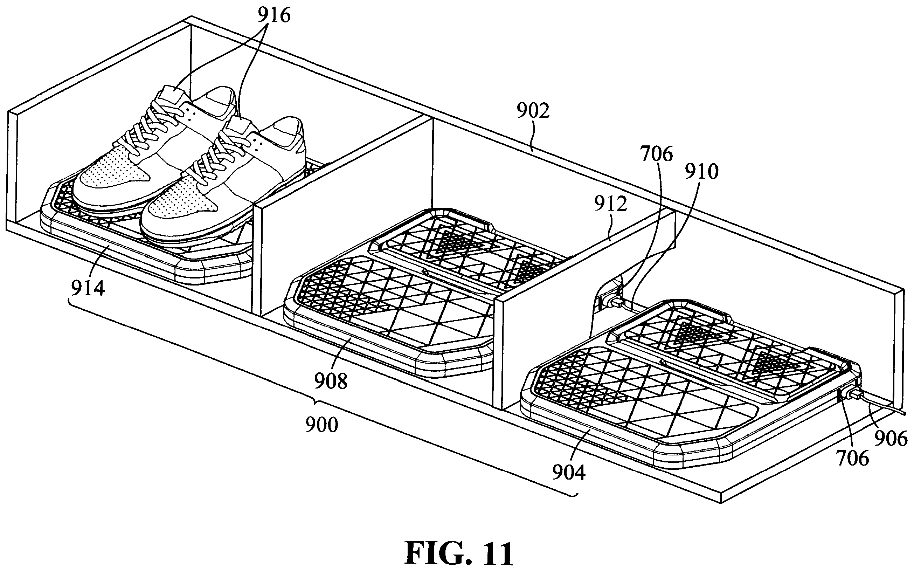

A plurality of charging mats 900 may be disposed in a side-by-side arrangement as shown in Figure 11. Referring to Figure 11 the charging mats 900 are each sized to fit within a gym locker or locker room cubical, portions of which are shown in cut away view at 902 in Figure 11. A first charging mat 904 is connected to a power source via a cable and connector 906. A second charging mat 908 has its first connector 706 connected via a cable and connector 910 to the second connector 712 (see Figure 8) of the first charging mat 904. The first charging mat 904 is separated by a partition 912 (shown partially cut away in Figure 11) from the second charging mat 908 and the cable and connector 910 passes through the partition. A third charging mat 914 is similarly connected to the second charging mat 908. Since the first connector 706 and second connector 712 (see Figure 8) for each mat are connected in parallel, the charging current supplied to the first charging mat 904 by the cable and connector 906 is thus also delivered to the second charging mat 908 and third charging mat 914.

A pair of shoes 916 disposed on the third charging mat 914 will thus be charged by the current supplied by the cable 906 through the first charging mat 904 and through the second charging mat 908. The power source feeding the plurality of charging mats 900 through the cable and connector 906 will thus need to be rated to supply sufficient charging current for charging multiple pairs of shoes simultaneously.

The placement of the charging mats 900 in the cubicles 902 allows for convenient storage of shoes 916 while simultaneously charging energy storage devices 204 within the respective insoles 200 of the shoes. The housing 102 of the charging apparatus 100 is sized and configured to permit removal from the charging mat for use separate from the charging mat when the user of the mat needs to be away from the locker room for a period of time. The battery 310 of the charging apparatus 100 should generally be fully charged by the charging current supplied via the cable 906 and should thus have capacity to charge the shoes multiple times when the charging apparatus 100 is removed. If the battery 310 in the charging apparatus 100 becomes depleted, a separate charging supply can be connected to the charge connector 312 for recharging.

Referring to Figure 12, a kiosk system in accordance with another disclosed embodiment is shown generally at 1000. The kiosk system 1000 includes a base 1002 and a column 1004 supporting a plurality of shelves 1006 in a vertically spaced apart arrangement. The kiosk system 1000 also includes a host controller 1008 housed within the column 1004 (shown in cut away view) and an interactive display 1010 located at the top of the column for displaying operating information to a user and for receiving user input. Each shelf of the plurality of shelves 1006 has a charging apparatus 100 disposed on the shelf and connected to the host controller 1008 for receiving a charging current and for controlling the charging apparatus 100. The kiosk system 1000 receives operating power via a mains power cable 1012. Each shelf of the plurality of shelves 1006 is able to receive a pair of insoles 1014 for charging.

The kiosk system 1000 thus has locations for accommodating a plurality of insoles 1014 of differing sizes. As described above in connection with the insole 200 shown in Figure 2, each insole 200 has the energy storage device 204, circuitry 206, and receiving antenna 208.

A block diagram of the host controller 1008 is shown in Figure 13. Referring to Figure 13, the host controller 1008 includes a controller unit 1100, such as an industrial PC or other microprocessor based controller having a microprocessor 1102 and a memory 1104. Program codes for directing the microprocessor 1106 to carry out various functions are stored in the program memory 1104, which may be implemented as a random access memory (RAM) and/or a hard disk drive (HDD), or a combination thereof.

The controller unit 1100 may be partly or fully implemented using a hardware logic circuit including discrete logic circuits, an application specific integrated circuit (ASIC), and/or a field-programmable gate array (FPGA).

The controller unit 1100 also includes a wireless interface 1106 having a wireless antenna 1108. The wireless interface 1106 may implement one or more wireless protocols, such as Bluetooth and/or IEEE 802.11 Wi-Fi protocol. The controller unit 1100 also includes a wired communications interface 1110 for connecting to a network 1112 via a high bandwidth communications channel. The network 1112 may be a local area network or a wide area network such as the internet. The wired communications interface 1110 is implemented as an Ethernet interface for connecting to the network 1112.

The controller unit 1100 also includes a display port 1114 for connecting to the interactive display 1010, which is operable to display a user interface and receive input information related to the test subject. The interactive display 1010 may be a touchscreen display for receiving and transmitting user input back to the controller unit 1100 via the display port 1114. The interactive display 1010 may be implemented using a tablet computer, which may connect to the controller unit 1100 either via the display port 1114, or wirelessly via the wireless antenna 1108 and wireless interface 1106.

The host controller 1008 also includes a power supply 1116 that receives an AC power feed via the mains power cable 1012 and generates voltages suitable for powering the controller unit 1100, the interactive display 1010, and the charging apparatuses 100. In the figure the host controller 1008 also includes an uninterruptible power supply (UPS) or battery 1118 that provides backup power when the kiosk system 1000 is not connected to a mains outlet via the mains power cable 1012.

Each charging apparatus 100 in the kiosk system 1000 is connected to the power supply 1116 for receiving a charging current and further connected via a signal line 1120 that carries communications signals between the wired communications interface 1110 of the controller unit 1100 and the charging apparatus 100 for communicating commands and status information between the controller unit 1100 and each charging apparatus 100. The communications interface of the controller unit 1100 thus also implements a protocol for wired communication with the respective charging apparatuses 100 associated with each location (charging apparatus 100) to determine a status associated with the location. The communication signals may be in accordance with the RS-485 protocol. Examples of the status associated with each charging apparatus 100 may be whether an insole 200 is present at the location, a size of the insole 200 present at the location, and a state of charge of insole 200 stored at the location.

The controller unit 1100 is able to wirelessly connect via the wireless interface 1106 to the embedded circuitry 206 in the insole 200 for receiving movement information from the insole 200 while being worn in a test subject's shoes during a movement test. Each pair of insoles 200 communicates to the controller unit 1100 using a wireless data channel 1125 specific to the pair of insoles 200, in other words, discrete data channels 1125. In one embodiment the embedded circuitry 206 in the insole 200 may include a plurality of inertial sensors 1124 and 1126 that generate signals representing movement of the insoles worn in the test subject's shoes. During movement of the test subject's shoes, each insole 200 may generate data representing an instantaneous three axis position and heading defining the trajectory of the insole in space. Capturing the inertial movement data requires a relatively high transmission bandwidth, which while possible over an IEEE 802.11 connection, would generally not be at all practical over a Bluetooth connection. The IEEE 802.11 protocol implemented by the wireless interface 1106 and embedded circuitry 206 will generally have sufficient bandwidth to receive continuous movement information from each of the plurality of inertial sensors 1124, 1126 in each of the insoles 200 during the movement test.

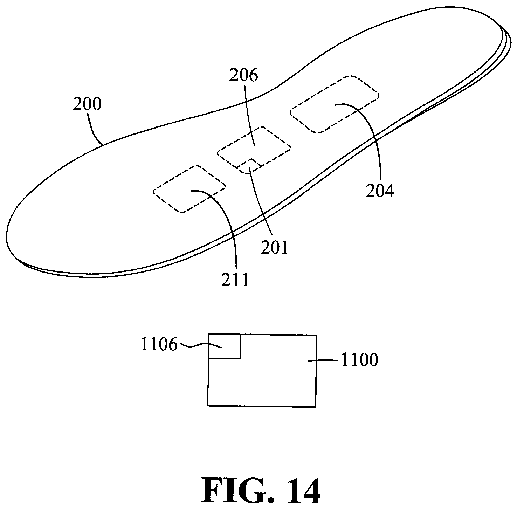

As shown in Figure 14, the embedded circuitry 206 in the insole 200 may include a buffer memory 201 for accumulating movement information. The wireless interface 211 in the insole 200 may be operably configured to periodically transmit movement information essentially in real time to the wireless interface 1106 of the controller unit 1100 from the buffer memory 201. The movement information (data) are transmitted through a discrete data channel - one for each insole of a plurality of insoles, such that multiple insoles may be transmitting data concomitantly. Thereafter, the wireless interface 211 of the insole 200 may be placed in a standby mode to conserve energy. IEEE 802.11 communications, while providing high transmission bandwidth, consume more energy than Bluetooth communications and if permitted to continuously transmit movement data may end up draining the energy storage device 204 of the insole 200.

The wireless interface 1106 of the controller unit 1100 and the wireless interface of the insole 200 each further implement a low power wireless protocol (for example, a Bluetooth protocol) for transfer of data other than the movement data between the embedded circuitry 206 and the controller unit. The low power protocol provides for energy efficient transmission of data that does not require high bandwidth. The data includes but is not limited to: 1. Contact time with the ground to predict fatigue (or even disease); 2. Data relating to fatigue prediction (athlete, soldier). The system determines an initial ground contact baseline as the person walks, runs, jumps, etc. when s/he is healthy/energized (e.g. at the beginning of the day, the beginning of the week, preseason, etc.). Over time, as the person gets tired/fatigued, the gate changes and there is more contact between the person's foot & the ground. The system acquires the data for each person in a group (e.g. sports team, military group) and provides reports about the group as a whole, or individuals within the group who are more fatigued than the other members, etc.; and 3. Early disease detection/prediction via gait changes over time (e.g. neurological disorders where gait changes signal disease progression... e.g. Parkinson's shuffling).

Movement data received from the insoles 200 at the wireless interface 1106 of the controller unit 1100 may be analyzed on the controller unit 1100 and results displayed on the interactive display 1010. Alternatively, the movement data may be transferred via the wired communications interface 1110 over the network 1112 to a networked computing resource 1128, such as an Amazon^{®} Web Services (AWS) or Google^{®} Cloud, cloud computing platform. The networked computing resource 1128 may be operably configured to receive and process the movement data and to make movement test results available to the test subject. Both the microprocessor 1102 of the controller unit 1100 and the networked computing resource 1128 are configured to have sufficient processing power to effectively perform the movement data processing. The test results produced by the microprocessor 1102 and networked computing resource 1128 may be saved to a network location for access by an internet browser over an internet connection for viewing. Alternatively, the networked computing resource 1128 may transmit the results back to the controller unit 1100 via the network 1112, facilitating display of the movement test results on the interactive display 1010.

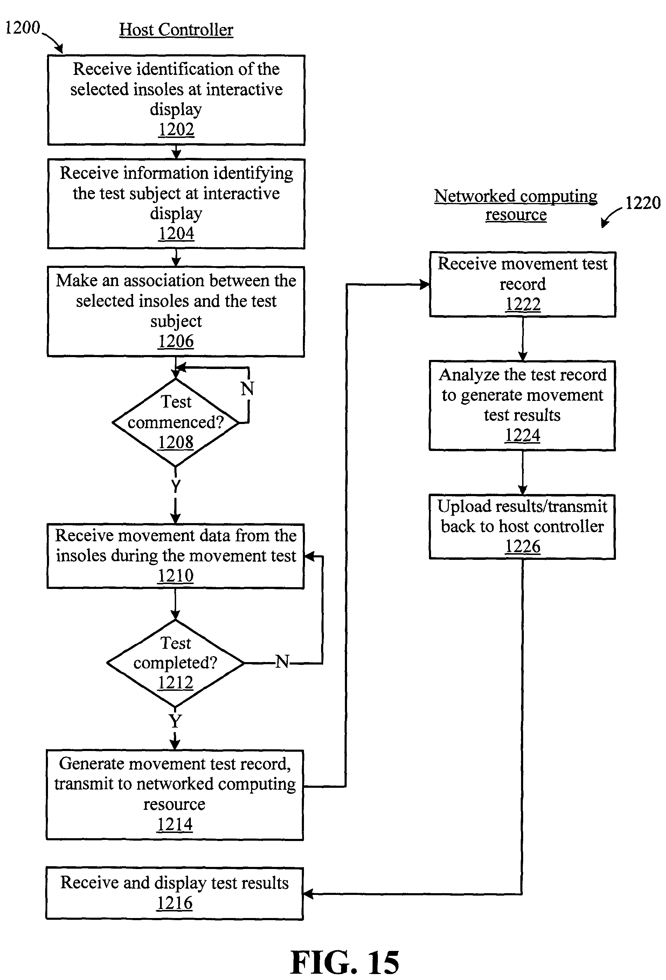

Referring to Figure 15, a flowchart depicting blocks of code for directing the processor circuit 1102 to conduct the movement test is shown general at 1200. The blocks generally represent codes that may be read from the memory 1104 for directing the microprocessor 1102 to perform various functions. The actual code to implement each block may be written in any suitable program language, such as C, C++, C#, Java, and/or assembly code, for example. The process begins when a test subject selects one of the sets of insoles from the kiosk system 1000. Block 1202 directs the microprocessor 1102 to receive an identification of the selected pair of insoles at the interactive display 1010. Identification of the selected insoles may take various forms. For example the test subject, other operator, or therapist may key in a number on the insoles, scan a code such as a QR code or barcode, or a status provided by the charging apparatus 100 may indicate that a specific insole pair has been removed from one of the plurality of shelves 1006. Alternatively, the insole pair is provided with a specific wireless data communication channel that automatically identifies the pair of insoles and tags the data being transmitted as being from that pair of insoles.

Block 1204 then directs the microprocessor 1402 to receive information identifying the test subject via the interactive display. For example, the test subject's name, height, weight, age, and other personal information may be entered into the interactive display 1010. These test subject identifiers may be associated with a specific user prior to them being provided with a specific pair of insoles. Block 1206 then directs the microprocessor 1402 to make an association between the selected insoles and the test subject. This step may be unnecessary if the pair of insoles has a wireless data communication channel that is specific to the given pair of insoles and the test subject has already been identified and associated with the pair of insoles. The microprocessor 1402 may implement a database in the memory 1104, the database including information related to various test subjects and the insoles located on the plurality of shelves 1006 of the kiosk system 1000. The association between the selected insoles and the test subject ensures that any movement test results received from the insoles are correctly attributed to the test subject.

Block 1206 then directs the microprocessor 1102 to block 1208, where the microprocessor is directed to wait until the test commences. For example, the microprocessor 1102 may cause a start button to be displayed on the interactive display 1010, which can be actuated to indicate that the controller unit 1100 should receive movement data from the insole. In other embodiments, the interactive display 1010 may prompt the test subject through a series of specific movement tests. When the test commences at block 1208, block 1210 directs the microprocessor to receive movement data from the insoles. As disclosed above the movement data may be transferred via an IEEE 802.11 wireless protocol transmission and received by the wireless interface 1106 of the controller unit 1100. Block 1210 then directs the microprocessor to determine whether the movement test has completed. If the test is not yet completed, block 1212 directs the microprocessor 1102 back to block 1210 for receiving further movement data. If at block 1212 the test is completed the microprocessor 1102 is directed to block 1214 where a movement test record is generated. The movement test record includes movement data and data identifying the test subject. Block 1214 then transmits the movement test record via the wired communications interface 1110 and network 1112 to the networked computing resource 1128.

A flowchart depicting processing of the movement test record by the networked computing resource 1128 is shown at 1220 in Figure 15. The networked computing resource 1128 receives the movement test record at block 1222, and analyses the test record at block 1224 to generate movement test results. At block 1226 the networked computing resource 1128 then uploads the test results to a web page on the internet (i.e. a location on the network 1112) and may also transmit the test results or a link to a test result web page back to the controller unit 1100.

The process 1200 then continues at block 1216 when the controller unit 1100 receives the test results or link at the wired communications interface 1110. Block 1216 may direct the microprocessor 1102 to display the results on the interactive display 1010 for viewing by the test subject.

The above disclosed embodiments provide a convenient infrastructure for charging an energy storage element in a shoe or insole, whether in a gym or locker room or away on the road. The charging apparatus 100 is small enough to easily pack and the included battery provides sufficient capacity for multiple charges while the charging apparatus is not inserted in the charging mat 700 and receiving charging current. Should the charging apparatus 100 require charging, a commonly available charging connector and source may be used to charge the internal battery.

The kiosk system 1000 further provides an infrastructure for administering a movement test using a selected insole from one of the kiosk locations. For example, the kiosk system 1000 may be located in a physiotherapist's office and used to perform movement tests on patients. Alternatively, the kiosk system 1000 may be located in a gym or a sports team locker room for use by athletes.

## Claims

1. A system for collecting, storing and analyzing movement data associated with physical activity, the system comprising:
at least one insole (200, 750, 751) for use with an energy storage device (204, 754), the insole including: circuitry (206, 756), which includes a memory (201, 764), a processor (762) under control of the memory, and a first polarity magnet or magnetic material (766, 768);
at least one dock (772), which includes a second polarity magnet or a magnetic material (776) which is magnetically attracted to the first polarity magnet or magnetic material (766, 768) of the insole (750, 751);
at least one sensor (759), which is at least one of a motion sensor and a pressure sensor and which is in electronic communication with the processor (762);
a charger (758) for electrical communication with an energy storage device (754); an electronic communicator (800), which is in electronic communication with the circuitry (756) and is configured for electronic communication with the dock (772); and
a substrate (752), which retains the circuitry (756), the sensor (759), the charger (758) and the electronic communicator (800), the dock (772) including an electronic communicator for electronic communication with the insole (750, 751) and for electronic communication with a computer, wherein the memory is configured to instruct the processor (762) to collect data from the sensor to provide a data set; configured to store the data set; and configured to instruct the processor, in response to the magnetic attraction between the first polarity magnet or magnetic material (766, 768) and the second polarity magnet or magnetic material (776), to download the data set to the dock.

2. The system of claim 1, wherein the electronic communicators (800) are a wireless interface.

3. The system of claim 2, the system comprising at least a first insole (750) and a second insole (751), the wireless interface of each insole having a discrete data channel (1125).

4. The system of claim 3, wherein the memory (764) is further configured to send the data set periodically during data collection via the discrete data channel (1125) of each insole (750, 751) to the dock (772).

5. A method of collecting and storing movement data, the method comprising:
- a user selecting at least one insole (750, 751) from a dock (772), the insole including circuitry (206, 756), which includes a memory (201, 764), a processor (762), the processor under control of the memory, and a first polarity magnet or magnetic material (766, 768); the dock including a communications interface (1110) and a second polarity magnet or magnetic material (776) which is magnetically attracted to the first polarity magnet or magnetic material; at least one sensor (759), which is at least one of a motion sensor and a pressure sensor and which is in electronic communication with the processor; an energy storage device (204, 754), which is in electrical communication with the circuitry; a charger (758) in electrical communication with the energy storage device; an insole communications interface (1110), which is in electronic communication with the circuitry and the dock; and a substrate (752), which retains the circuitry, the sensor, the charger and the communications interface;
- the user removing the insole (750, 751) from the dock (772) and releasably retaining the insole on the user's foot;
- the processor (762) signaling the memory (764) to start data collection;
- the sensor (759) sending data to the circuitry (756), in response to a magnetic attraction between the first polarity magnet (766, 768) and the second polarity magnet (776), to provide a data set; and
- the memory (764) storing the data set.

6. The method of claim 5, wherein the removing the insole (750, 751) from the dock (772) breaks a magnetic field between the first polarity magnet or magnetic material (766, 768) of the insole and the second polarity magnet or magnetic material (776) of the dock (772) and causing a switch (770) under the control of the processor (762) in the insole to start data collection.

7. The method of claim 5 or 6, further comprising:
- the user removing the insole (750, 751) and returning it to the dock (772) resulting in the magnetic field between the first and second polarity magnets or magnetic materials (766, 768, 776) of the insole and the dock, respectively, for causing the switch (770) to start downloading the data from the insole to the dock.

8. The method of claim 7, further comprising:
- the dock (772) transmitting the data set to a computer; and
- the computer analyzing the data set to provide a predictive model of an analyzed data set.

## Patentansprüche

1. System zum Erfassen, Speichern und Analysieren von mit körperlicher Aktivität assoziierten Bewegungsdaten, wobei das System Folgendes umfasst:
mindestens eine Innensohle (200, 750, 751) zur Verwendung mit einer Energiespeichervorrichtung (204, 754), wobei die Innensohle Folgendes enthält: eine Schaltungsanordnung (206, 756), enthaltend einen Speicher (201, 764), einen Prozessor (762), der durch den Speicher gesteuert wird, und einen Magnet oder ein Magnetmaterial (766, 768) mit einer ersten Polung;
mindestens eine Dockingeinrichtung (772), die einen Magnet oder ein Magnetmaterial (776) mit einer zweiten Polung, der/das von dem Magnet oder Magnetmaterial (766, 768) der Innensohle (750, 751) mit einer ersten Polung magnetisch angezogen wird, enthält;
mindestens einen Sensor (759), der mindestens einer von einem Bewegungssensor und einem Drucksensor ist und der in elektronischer Kommunikation mit dem Prozessor (762) steht;
eine Ladeeinrichtung (758) zur elektrischen Kommunikation mit einer Energiespeichervorrichtung (754); eine elektronische Kommunikationseinrichtung (800), die in elektronischer Kommunikation mit der Schaltungsanordnung (756) steht und zur elektronischen Kommunikation mit der Dockingeinrichtung (772) konfiguriert ist; und
ein Trägermaterial (752), das die Schaltungsanordnung (756), den Sensor (759), die Ladeeinrichtung (758) und die elektronische Kommunikationseinrichtung (800) hält, wobei die Dockingeinrichtung (772) eine elektronische Kommunikationseinrichtung zur elektronischen Kommunikation mit der Innensohle (750, 751) und zur elektronischen Kommunikation mit einem Computer enthält, wobei der Speicher dazu konfiguriert ist, den Prozessor (762) dazu anzuweisen, Daten von dem Sensor zu erfassen, um einen Datensatz bereitzustellen, dazu konfiguriert ist, den Datensatz zu speichern, und dazu konfiguriert ist, den Prozessor dazu anzuweisen, als Reaktion auf die magnetische Anziehung zwischen dem Magnet oder Magnetmaterial (766, 768) mit einer ersten Polung und dem Magnet oder Magnetmaterial (776) mit einer zweiten Polung den Datensatz in die Dockingeinrichtung herunterzuladen.

2. System nach Anspruch 1, wobei die elektronischen Kommunikationseinrichtungen (800) eine drahtlose Schnittstelle sind.

3. System nach Anspruch 2, wobei das System mindestens eine erste Innensohle (750) und eine zweite Innensohle (751) umfasst, wobei die drahtlose Schnittstelle jeder Innensohle einen diskreten Datenkanal (1125) aufweist.

4. System nach Anspruch 3, wobei der Speicher (764) ferner dazu konfiguriert ist, den Datensatz periodisch während der Datenerfassung über den diskreten Datenkanal (1125) jeder Innensohle (750, 751) an die Dockingeinrichtung (772) zu senden.

5. Verfahren zum Erfassen und Speichern von Bewegungsdaten, wobei das Verfahren Folgendes umfasst:
- durch einen Benutzer Auswählen mindestens einer Innensohle (750, 751) von einer Dockingeinrichtung (772), wobei die Innensohle eine Schaltungsanordnung (206, 756), enthaltend einen Speicher (201, 764), einen Prozessor (762), wobei der Prozessor durch den Speicher gesteuert wird, und einen Magnet oder ein Magnetmaterial (766, 768) mit einer ersten Polung enthält; wobei die Dockingeinrichtung eine Kommunikationsschnittstelle (1110) und einen Magnet oder ein Magnetmaterial (776) mit einer zweiten Polung, der/das von dem Magnet oder Magnetmaterial mit einer ersten Polung magnetisch angezogen wird, enthält; mindestens einen Sensor (759), der mindestens einer von einem Bewegungssensor und einem Drucksensor ist und der in elektronischer Kommunikation mit dem Prozessor steht; eine Energiespeichervorrichtung (204, 754), die in elektrischer Kommunikation mit der Schaltungsanordnung steht; eine Ladeeinrichtung (758) in elektrischer Kommunikation mit der Energiespeichervorrichtung; eine Innensohlenkommunikationsschnittstelle (1110), die in elektronischer Kommunikation mit der Schaltungsanordnung und der Dockingeinrichtung steht; und ein Trägermaterial (752), das die Schaltungsanordnung, den Sensor, die Ladeeinrichtung und die Kommunikationsschnittstelle hält;
- durch den Benutzer Entfernen der Innensohle (750, 751) von der Dockingeinrichtung (772) und lösbares Aufbringen der Innensohle auf dem Fuß des Benutzers;
- durch den Prozessor (762) Signalisieren an den Speicher (764), dass mit der Datenerfassung zu beginnen ist;
- durch den Sensor (759) Senden von Daten an die Schaltungsanordnung (756), als Reaktion auf eine magnetische Anziehung zwischen dem Magnet (766, 768) mit einer ersten Polung und dem Magnet (776) mit einer zweiten Polung, um einen Datensatz bereitzustellen; und
- durch den Speicher (764) Speichern des Datensatzes.

6. Verfahren nach Anspruch 5, wobei durch das Entfernen der Innensohle (750, 751) von der Dockingeinrichtung (772) ein Magnetfeld zwischen dem Magnet oder Magnetmaterial (766, 768) der Innensohle mit einer ersten Polung und dem Magnet oder Magnetmaterial (776) der Dockingeinrichtung (772) mit einer zweiten Polung unterbrochen wird, und Bewirken, dass durch einen Schalter (770) gesteuert durch den Prozessor (762) in der Innensohle mit der Datenerfassung begonnen wird.

7. Verfahren nach Anspruch 5 oder 6, das ferner Folgendes umfasst:
- durch den Benutzer Entfernen der Innensohle (750, 751) und Zurückbewegen dieser zu der Dockingeinrichtung (772), wodurch sich das Magnetfeld zwischen den Magneten oder Magnetmaterialien (766, 768, 776) der Innensohle und der Dockingeinrichtung mit einer ersten bzw. einer zweiten Polung ergibt, um zu bewirken, dass durch den Schalter (770) mit dem Herunterladen der Daten von der Innensohle in die Dockingeinrichtung begonnen wird.

8. Verfahren nach Anspruch 7, das ferner Folgendes umfasst:
- durch die Dockingeinrichtung (772) Übertragen des Datensatzes an einen Computer; und
- durch den Computer Analysieren des Datensatzes, um ein prädiktives Modell eines analysierten Datensatzes bereitzustellen.

## Revendications

1. Système permettant de collecter, de stocker et d'analyser des données de mouvement associées à une activité physique, le système comprenant :
au moins une semelle intérieure (200, 750, 751) destinée à être utilisée avec un dispositif de stockage d'énergie (204, 754), la semelle intérieure comprenant : une circuiterie (206, 756), qui comprend une mémoire (201, 764), un processeur (762) sous commande de la mémoire, et un aimant ou un matériau magnétique de première polarité (766, 768) ;
au moins une station d'accueil (772), qui comprend un aimant ou un matériau magnétique de seconde polarité (776) qui est attiré magnétiquement par l'aimant ou le matériau magnétique de première polarité (766, 768) de la semelle intérieure (750, 751) ;
au moins un capteur (759), qui est un capteur de mouvement et/ou un capteur de pression et qui est en communication électronique avec le processeur (762) ;
un chargeur (758) destiné à être en communication électrique avec un dispositif de stockage d'énergie (754) ; un module de communication électronique (800), qui est en communication électronique avec la circuiterie (756) et qui est configuré pour communiquer électroniquement avec la station d'accueil (772) ; et
un substrat (752) qui retient la circuiterie (756), le capteur (759), le chargeur (758) et le module de communication électronique (800), la station d'accueil (772) comprenant un module de communication électronique pour communiquer électroniquement avec la semelle intérieure (750, 751) et pour communiquer électroniquement avec un ordinateur, la mémoire étant configurée pour donner l'instruction au processeur (762) de collecter des données auprès du capteur pour fournir un ensemble de données ; configurée pour stocker l'ensemble de données ; et configurée pour donner l'instruction au processeur, en réponse à l'attraction magnétique entre l'aimant ou le matériau magnétique de première polarité (766, 768) et l'aimant ou le matériau magnétique de seconde polarité (776), de télécharger l'ensemble de données sur la station d'accueil.

2. Système selon la revendication 1, les modules de communication électroniques (800) étant une interface sans fil.

3. Système selon la revendication 2, le système comprenant au moins une première semelle intérieure (750) et une seconde semelle intérieure (751), l'interface sans fil de chaque semelle intérieure comportant un canal de données discret (1125).

4. Système selon la revendication 3, la mémoire (764) étant en outre configurée pour envoyer périodiquement l'ensemble de données, pendant la collecte de données, par l'intermédiaire du canal de données discret (1125) de chaque semelle intérieure (750, 751), à la station d'accueil (772).

5. Procédé de collecte et de stockage de données de mouvement, le procédé comprenant les étapes consistant à :
- sélectionner, par un utilisateur, au moins une semelle intérieure (750, 751) à partir d'une station d'accueil (772), la semelle intérieure comprenant une circuiterie (206, 756), qui comprend une mémoire (201, 764), un processeur (762), le processeur étant sous commande de la mémoire, et un aimant ou un matériau magnétique de première polarité (766, 768) ; la station d'accueil comprenant une interface de communication (1110) et un aimant ou un matériau magnétique de seconde polarité (776) qui est attiré magnétiquement par l'aimant ou le matériau magnétique de première polarité ; au moins un capteur (759), qui est un capteur de mouvement et/ou un capteur de pression et qui est en communication électronique avec le processeur ; un dispositif de stockage d'énergie (204, 754), qui est en communication électrique avec la circuiterie ; un chargeur (758) en communication électrique avec le dispositif de stockage d'énergie ; une interface de communication de semelle intérieure (1110), qui est en communication électronique avec la circuiterie et la station d'accueil ; et un substrat (752), qui retient la circuiterie, le capteur, le chargeur et l'interface de communication ;
- retirer, par l'utilisateur, la semelle intérieure (750, 751) de la station d'accueil (772) et retenir amovible la semelle intérieure sur le pied de l'utilisateur ;
- signaler, par le processeur (762), à la mémoire (764) de démarrer la collecte de données ;
- envoyer, par le capteur (759), des données à la circuiterie (756), en réponse à une attraction magnétique entre l'aimant de première polarité (766, 768) et l'aimant de seconde polarité (776), pour fournir un ensemble de données ; et
- stocker, par la mémoire (764), l'ensemble de données.

6. Procédé selon la revendication 5, dans lequel le retrait de la semelle intérieure (750, 751) de la station d'accueil (772) rompe un champ magnétique entre l'aimant ou le matériau magnétique de première polarité (766, 768) de la semelle intérieure et l'aimant ou le matériau magnétique de seconde polarité (776) de la station d'accueil (772), et amenant un commutateur (770) sous commande du processeur (762) dans la semelle intérieure à démarrer la collecte de données.

7. Procédé selon la revendication 5 ou 6, comprenant en outre l'étape consistant à :
- retirer, par l'utilisateur, la semelle intérieure (750, 751) et la remettre en place sur la station d'accueil (772), de manière à créer le champ magnétique entre les aimants ou les matériaux magnétiques de première et seconde polarités (766, 768, 776) respectivement de la semelle intérieure et de la station d'accueil, afin d'amener le commutateur (770) à démarrer le téléchargement des données de la semelle intérieure sur la station d'accueil.

8. Procédé selon la revendication 7, comprenant en outre les étapes consistant à :
- émettre, par la station d'accueil (772), l'ensemble de données vers un ordinateur ; et
- analyser, par l'ordinateur, l'ensemble de données pour fournir un modèle prédictif d'un ensemble de données analysé.
